# EUROPEAN PATENT APPLICATION

(11) **EP 3 263 044 A1**
(43) Date of publication of application: **03.01.2018**
(21) Application number: 16755241.3
(22) Date of filing: 12.02.2016
(51) Int. Cl.: A61B 17/06

(54) **PACKAGING MATERIAL FOR SUTURE THREAD WITH NEEDLE**

(30) Priority: 27.02.2015 JP 2015038434
(71) Applicant: Mani, Inc., Tochigi 321-3231 (JP)
(72) Inventor: OGINO, Masamitsu, Utsunomiya-shi Tochigi 321-3231 (JP); KATOH, Kazuaki, Utsunomiya-shi Tochigi 321-3231 (JP); SHINOZAKI, Akihiro, Utsunomiya-shi Tochigi 321-3231 (JP)
(74) Representative: Isarpatent
(86) International application number: PCT/JP2016/054080
(87) International publication number: WO 2016/136494

(57) **Abstract**

There is provided a packaging material for a suture thread with needle allowing extraction of the suture thread with needle from the packaging material without the suture thread being entangled, and making it difficult for the suture thread to be scraped at a point of the packaging material when extracting the suture thread with needle stored in the packaging material. The packaging material (10) that is folded in three or more for storing the suture thread with needle (30) includes a curved cutout part (20) on an end part of the packaging material (10) in an open state. A through-hole (25) for the suture thread (32) is formed by a crease and the curved cutout part (20) when the packaging material is folded so that the curved cutout part (20) is positioned on the inner side of the packaging material (10), and the suture thread (32) passes through the through-hole (25) when extracting the stored suture thread with needle (30) from the packaging material (10).

## Description

### Technical Field

The present invention relates to a packaging material for storing a suture thread with needle.

### Background Art

A suture thread with needle used in ophthalmology, dentistry, etc. is sold stored in a packaging material made of paper, resin, etc. or a resin case. The suture thread with needle is often sold stored in a packaging material made of paper, resin, etc. rather than a resin case with consideration of cost performance etc. since it is used only one time.

The suture thread with needle stored in a packaging material is used after extracting the suture thread from the packaging material, with the suture needle held using a needle holder, so that the suture needle and the suture thread is not contaminated with bacteria etc. before use. When the suture thread entangles within the packaging material and is difficult to extract at this time, the suture thread may be pulled out from the suture needle, or the suture needle may be extracted from the packaging material with the suture thread sticking to the suture needle. Then, the packaging material may be structured having a hole such as an opening such that the suture thread can be pulled out through that hole, thereby making it easier to eliminate entangling of the suture thread and to extract it.

However, if the suture thread is scraped at a point such as a crease edge of the packaging material when extracting the suture thread through the hole such as an opening in the packaging material, a problem that the suture thread is damaged may occur, or in the case where the packaging material is made of paper, the packaging material may be damaged and paper dust may adhere to the suture thread.

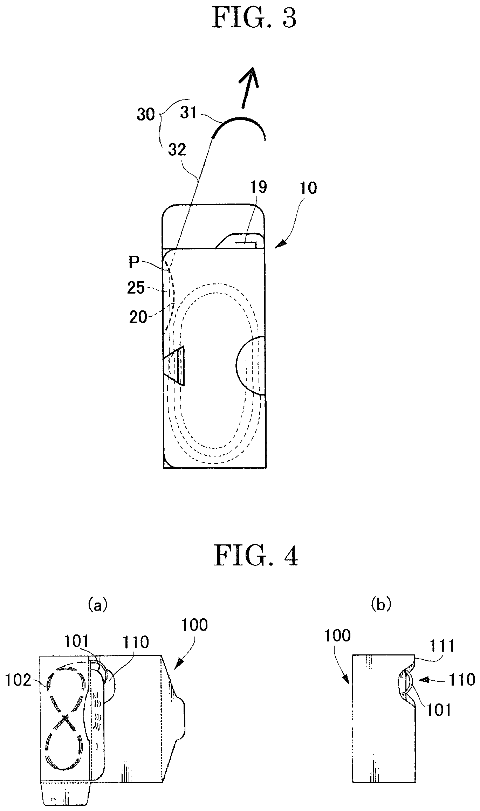

FIG. 4 is a top view of a conventional packaging material described in Patent Document 1; where FIG. 4(a) illustrates a state of the conventional packaging material during packaging, and FIG. 4(b) illustrates the state of the conventional packaging material after packaging. This packaging material 100 is structured such that a suture thread 102 is extracted from an opening 110 with a suture needle 101 grasped with a needle holder. However, since it is assumed with this configuration that the suture thread 102 will be scraped at a point on an end part 111 of the opening 110 during extraction, there are concerns that the suture thread 102 may be damaged, and that paper dust of the packaging material 100 may adhere to the suture thread 102.

### Prior Art Documents

### Patent Documents

[Patent Document 1] JP 3248705

### DISCLOSURE OF INVENTION

### Problem to be Solved by the Invention

In light of these problems, the present invention aims to provide a packaging material for a suture thread with needle, which allows extraction of the suture thread from the packaging material without the suture thread being entangled, and which makes it difficult for the suture thread to be scraped at a point of the packaging material when extracting the suture thread with needle stored in the packaging material.

### Solution to the Problem

A packaging material for a suture thread with needle of the present invention that is folded in three or more for storing a suture thread with needle includes a curved cutout part on an end part of the packaging material in an open state. A through-hole for the suture thread is formed by a crease and the curved cutout part when it is folded so that the curved cutout part is positioned on the inner side of the packaging material, and the suture thread passes through the through-hole when extracting the stored suture thread with needle from the packaging material.

When the suture thread and the curved cutout part may make contact and the suture thread is pulled out, the position of contact of the curved cutout part and the suture thread may move.

Moreover, the curved cutout part may have an arc shape, the packaging material may be folded in four, and a needle retainer part for applying pressure to the needle of the suture thread with needle may be provided.

### Advantageous Effect of the Invention

Use of the packaging material for the suture thread with needle of the present invention brings about beneficial effects that the suture thread is not easily entangled, and that damage to the suture thread and adherence of paper dust can be controlled when extracting the suture thread with needle from the packaging material.

### BRIEF DESCRIPTION OF THE DRAWINGS

FIG. 1 is a drawing explaining a process of storing a suture thread with needle in a packaging material according to the present invention;
FIG. 2 shows schematic cross-sections after packaging;
FIG. 3 is a diagram describing how to extract the suture thread with needle from the packaging material; and
FIG. 4 is a top view of a conventional packaging material; wherein FIG. 4(a) illustrates a state of the conventional packaging material during packaging, and FIG. 4(b) illustrates the state of the conventional packaging material after packaging.

### DESCRIPTION OF EMBODIMENTS

An embodiment according to the present invention is described below with reference to accompanying drawings.

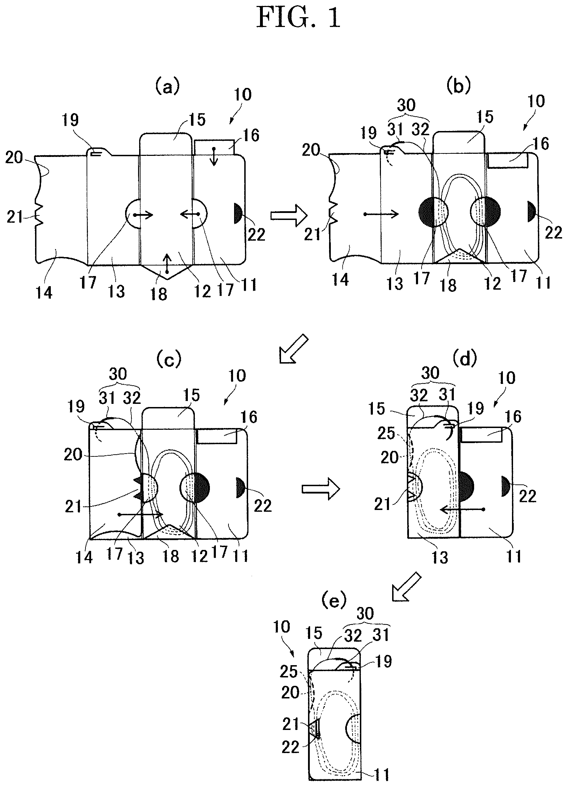

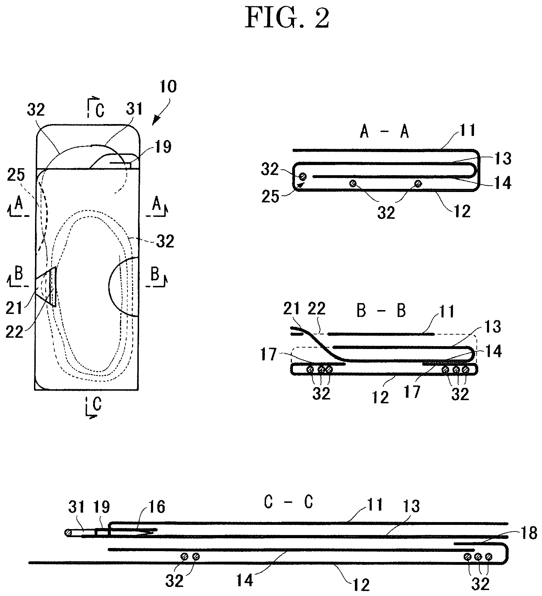

FIG. 1 is a drawing explaining a process of storing a suture thread with a needle in a packaging material according to the present invention. Arrow portions in the respective drawings are folded in order of FIGS. 1(a), 1(b), 1(c), 1(d), and 1(e) so as to store a suture thread with needle 30. FIG. 2 shows schematic cross-sections after packaging. The schematic cross-sections are drawings schematically illustrating in an easy to understand manner the packaging material 10 after packaging cut crosswise in two places (A-A cross section, B-B cross section) and cut lengthwise in one place (C-C cross section).

The packaging material 10 of the embodiment has such a four-fold structure as four large surfaces are folded and overlaid. Here, in the description of the embodiment, the four surfaces are a first surface 11, a second surface 12, a third surface 13, and a fourth surface 14 in order from the right side of FIG. 1(a). Note that double slits are formed in order to give a little thickness to the boundary between the first surface 11 and the second surface 12 and the boundary between the second surface 12 and the third surface 13 when folded.

The four surfaces are folded by first overlaying the fourth surface 14 on top of the third surface 13 (FIG. 1(c)), then overlaying the overlapped portion of the fourth surface 14 and the third surface 13 on top of the second surface 12 (FIG. 1(d)), and finally overlaying the first surface 11 on the top (FIG. 1(e)). In other words, the packaging material 10 after packaging has the first surface 11, the third surface 13, the fourth surface 14, and the second surface 12 laid on top of each other in this order from the top, as illustrated in the respective schematic cross-sections of FIG. 2. Roles etc. of each surface are described next.

The first surface 11 is provided with a needle retainer part 16 extending beyond the upper end. This needle retainer part 16 is folded so as to overlap the first surface 11, as indicated by an arrow in FIG. 1(a). The needle retainer part 16 has elasticity in the direction of restoring when folded, and the elasticity applies pressure on the suture needle 31 as illustrated in the C-C cross section of FIG. 2. As a result, movement of the suture needle 31 after packaging can be controlled. Note that since pressure is applied overall including the suture needle 31, it prevents the stored suture thread 32 from slipping out from the upper part of the packaging material 10.

Moreover, the first surface 11 includes a female locking part 22. The female locking part 22 is a hole cut out in a semicircle form according to the embodiment, and is used for preventing the folded packaging material 10 from opening on the condition that a male locking part 21 provided on the fourth surface 14 has passed through the hole and has hooked on to the female locking part, as illustrated in the B-B cross-section of FIG. 2. Note that the hole shape of the female locking part 22 is not particularly limited to a semicircle as long as the male locking part 21 can pass through and be hooked.

The second surface 12 is the bottom surface when packaged and is the portion on which the suture needle 32 is placed directly. A handle part 15 is provided in a form extending beyond the upper end of the second surface 12. For easy extraction of the suture thread with needle 30 from the packaging material 10, using a needle holder to hold the suture thread with needle, the handle part 15 has a slit formed at the boundary with the second surface 12 such that it can be bent on the back surface side.

Furthermore, the second surface 12 includes thread retainer parts 17 and 17. The thread retainer parts 17 and 17 result from making arc-shaped slits in the first surface 11 and the third surface 13, and can hold the suture thread 32 down, as illustrated in the B-B cross-section of FIG. 2, by folding at the boundaries between the second surface 12 and the first surface 11 and between the second surface 12 and the third surface 13 toward the second surface 12. Provision of the thread retainer parts 17 and 17 allows prevention of the thread from spreading out when setting the suture thread on the second surface 12, and also prevention of the suture thread 32 from moving within the packaging material 10. Note that the form of the thread retainer parts 17 is not particularly limited to an arc shape as long as it can hold the suture thread 32 down.

Moreover, a bottom part 18 is provided in a form extending beyond the bottom end of the second surface 12. The bottom part 18 is a part folding back toward the second surface 12, and it not only holds the suture thread 32 down, but also keeps the suture thread 32 from sliding downward when holding the packaging material 10 with the handle part 15 upward. While the form of the bottom part 18 is an isosceles triangle according to the embodiment, it is not particularly limited to this shape. However, making the lower end of the fourth surface 14 a loose arc shape according to the embodiment results in such a structure as the vicinity of the isosceles triangle of the bottom part 18 is sandwiched between the fourth surface 14 and the third surface 13.

The third surface 13 includes a needle fastener part 19. The needle fastener part 19 has a cutout part formed in a lateral U-shape such that it extends slightly over the upper end of the third surface 13 so as to sandwich and hold the suture needle 31. Note that the shape of the cutout part is not particularly limited, as long as it is shaped capable of holding the tip of the approximately arc-shaped suture needle 31 at an angle facing the third surface 13 from the needle fastener part 19. If the suture needle 31 is held at such an angle, pressure from the needle retainer 16 provided on the first surface 11 will be applied to the tip of the suture needle 31. Moreover, the needle fastener part that is passed through the suture needle 31 as often seen with conventional packaging materials is effective in protecting the needle tip; however, in order to minimize reduction of sharpness so as to maintain the sharpness, a shape sandwiching near the middle of the suture needle 31 as in the embodiment is preferred.

The fourth surface 14 elastically presses against the suture thread 32 from above when folded, and includes a male locking part 21 and a curved cutout part 20. The male locking part 21 is a part for piercing and hooking to a female locking part 22 of the first surface 11 so as to keep the packaging material 10 from opening. The embodiment includes such a structure as the thread retainer part 17 makes the male locking part 21 appear precisely at the hole opened in the third surface 13 when the fourth surface 14 is folded. That is, the male locking part 21 is passed through two holes: the hole that is opened in the third surface 13 by the thread retainer part 17, and the female locking part 22 of the first surface 11, so as to lock, and thereby has an effect of raising the adhesion of the respective surfaces of the packaging material 10. As a result, in cooperation with the elastic action of the fourth surface 14, it is capable of maintaining the same shape of the suture thread 32 as it is set, and stably holding it, thereby preventing entanglement of the suture thread 32.

The curved cutout part 20 is provided on the left side end of the fourth surface 14. The curved cutout part 20 along with the crease at the boundary between the second surface 12 and the third surface 13 forms a through-hole 25 when folded. The through-hole 25 is a hole connecting a space sandwiched between the second surface 12 and the fourth surface 14 and a space sandwiched between the fourth surface 14 and the third surface 13, as illustrated in the A-A cross-section of FIG. 2. The through-hole 25 is a hole through which the suture thread 32 passes when extracting the suture thread with needle 30 from the packaging material 10, thereby allowing extraction without entanglement of the suture thread 32.

According to the embodiment, the suture thread with needle 30 is stored in the packaging material 10 in such a manner that the suture thread 32 placed in the space sandwiched between the second surface 12 and the fourth surface 14 passes through the through-hole 25 and the space sandwiched between the fourth surface 14 and the third surface and then connects to the suture needle 31. That is, the suture thread 32 shown at the position of the through-hole 25 in the A-A cross-section of FIG. 2 illustrates the suture thread 32 passing through the through-hole 25 precisely.

For example, if the through-hole was a hole formed independently in the fourth surface 14, the storage operation would be complicated because the suture thread 32 needs to be passed through the entire through-hole either from the end or from the suture needle 31 side when storing the suture thread with needle 30. However, since the through-hole 25 of the embodiment is formed by the curved cutout part 20 and the crease at the boundary between the second surface 12 and the third surface 13, once the suture thread 32 is set to be sandwiched by the third surface 13 and the fourth surface 14, then merely folding it makes the suture thread 32 pass through the through-hole 25. That is, the structure forming the through-hole 25 using the curved cutout par 20 as in the embodiment allows easy storage of the suture thread with needle 30.

FIG. 3 is a diagram describing how to extract the suture thread with needle from the packaging material. When extracting the stored suture thread with needle 30, the suture needle 31 is held by a needle holder to extract the suture thread 32. At this time, as described before, the suture thread 32 is passed through the through-hole 25 and then extracted, thereby preventing entangled thread from leaving the packaging material 10.

If the suture needle 31 is pulled in the direction of the arrow in the drawing, the suture thread 32 touches the curved cutout part 20 at a contact point P. At this time, when the contact point P is always a specific point, a problem that the suture thread 32 is damaged, and/or that paper dust adheres to the suture thread 32 in the case where the packaging material is made of paper may occur. Therefore, making the curved shape of the curved cutout part 20 an arc shape with a large diameter is proposed. If it is an arc shape, the position of the contact point P is easily moved when the suture thread 21 is pulled out, thereby preventing damage to the suture thread 32 and adherence of paper dust. Note that while the curved shape of the curved cutout part 20 is preferably an arc shape with a large diameter, it is not particularly limited to only an arc shape since a beneficial effect of controlling damage to the suture thread 32 etc. may be brought about as long as it is a smooth curved shape allowing movement of the position of the contact point P.

Such a structure as the curved cutout part 20 is provided allowing the curved cutout part 20 and the crease to form the through-hole 25 according to the embodiment may provide such beneficial effects that storage of the suture thread with needle 30 is easy, that the suture thread 32 is not easily entangled when extracting the suture thread with needle 30 from the packaging material 10, and that damage to the suture thread 32 and adherence of paper dust can be controlled.

Note that while omitted from the drawings, a three-fold packaging material omitting the first surface may be considered as another embodiment. Even in this case, since the curved cutout part 20 and the through-hole 25 can be formed in the same manner as the four-fold structure, the same beneficial effects as with the four-fold structure of controlling damage to the suture thread 32 and adherence of paper dust may be brought about.

### Explanation of References

10: Packaging material
11: First surface
12: Second surface
13: Third surface
14: Fourth surface
15: Handle part
16: Needle retainer part
17: Thread retainer part
18: Bottom part
19: Needle fastener part
20: Curved cutout part
21: Male locking part
22: Female locking part
25: Through-hole
30: Suture thread with needle
31: Suture needle
32: Suture thread
P: Contact point of suture thread and curved cutout part

## Claims

1. A packaging material folded in three or more for storing a suture thread with needle, comprising:
a curved cutout part on an end part of the packaging material in an open state; wherein when the packaging material is folded so that the curved cutout part is positioned on the inner side of the packaging material, a through-hole for the suture thread is formed by the curved cutout part and a crease resulting from folding the packaging material, and
the suture thread passes through the through-hole when extracting the stored suture thread with needle from the packaging material.

2. The packaging material for a suture thread with needle of Claim 1, wherein when the suture thread and the curved cutout part make contact and the suture thread is pulled out, the position of contact of the curved cutout part and the suture thread moves.

3. The packaging material for a suture thread with needle of either Claim 1 or Claim 2, wherein the curved cutout part has an arc shape.

4. The packaging material for a suture thread with needle of either Claim 1 or Claim 2, wherein the packaging material is folded in four.

5. The packaging material for a suture thread with needle of any one of Claim 1 to Claim 4, wherein the packaging material includes a needle retainer part for applying pressure to the needle of the suture thread with needle.
